# EUROPÄISCHE PATENTSCHRIFT

(11) **EP 1 603 526 B1**
(45) Veröffentlichungstag und Bekanntmachung des Hinweises auf die Patenterteilung: **19.12.2007**
(21) Anmeldenummer: 04706626.1
(22) Anmeldetag: 30.01.2004
(51) Int. Cl.: A61Q 19/04, A61K 8/81

(54) **BRÄUNUNGSHILFEN**
TANNING AIDS
PRODUITS D'AIDE AU BRONZAGE

(30) Priorität: 14.03.2003 DE 10311641
(43) Veröffentlichungstag der Anmeldung: 14.12.2005
(73) Patentinhaber: Evonik Röhm GmbH, 64293 Darmstadt (DE)
(72) Erfinder: SCHOCKE, Kai-Olivier, 64291 Darmstadt (DE); SEELMANN, Peter, A-1238 Wien (AT); HILD, Uwe, 64283 Gross-Umstadt (DE); HÄFNER, Birgit, 64839 Münster (DE); MOLNAR, Gerald, A-2440 Gramatneusiedl (AT); AUER, Erich, A-2440 Gramatneusiedl (AT)
(86) Internationale Anmeldenummer: PCT/EP2004/000842
(87) Internationale Veröffentlichungsnummer: WO 2004/080435

(56) Entgegenhaltungen:
- EP-A- 0 919 222
- EP-A- 1 273 284
- US-A- 3 595 819

## Beschreibung

Die vorliegende Erfindung betrifft Bräunungshilfen, die einen Polymethylmethacrylat-Formkörper umfassen.

Eine leicht gebräunte Haut gilt als Zeichen für Urlaub und Gesundheit. Um die Haut zu bräunen werden üblicherweise Sonnenschutzcreme und dergleichen als Bräunungshilfe eingesetzt, um die Haut vor Schädigungen durch UV-Strahlung zu schützen. Nachteilig an diesen Hilfsmitteln ist, dass derartige Cremes bei empfindlichen Personen Allergien auslösen können. Des weiteren sind viele dieser Substanzen nicht wasserfest. Dem entsprechend werden sie beim Baden abgelöst, und anschließend nicht erneut aufgetragen. Durch diese Nachlässigkeit kann es leicht zu Schädigungen der Haut kommen.

Darüber hinaus sind Vorrichtungen bekannt, die mit Hilfe von eingebauten UV-Strahlern eine Bräunung der Haut bewirken. In diesem Zusammenhang sind auch Liegevorrichtungen für Solarienanwendungen aus PMMA bekannt, die hohe Mengen an UV-Stabilisatoren bzw. UV-Absorbern enthalten, um den Kunststoff vor Abbau durch UV-Strahlung zu schützen. Eine Bräunung mit Sonnenlicht ist hiermit jedoch nicht möglich. Nachteilig an derartigen Vorrichtungen ist der hohe Energieverbrauch der UV-Strahler. Darüber hinaus ist ein Betreiben dieser Anlagen im Freien nicht vorgesehen, so dass das Bräunen eher als langweilig empfunden wird.

In Anbetracht des hier angegebenen und diskutierten Standes der Technik war es mithin Aufgabe der vorliegenden Erfindung Bräunungshilfen anzugeben, mit deren Hilfe eine natürliche Bräunung der Haut mit Hilfe von Sonnenlicht erzielt werden kann, ohne das die Haut in Berührung mit Sonnenschutzcreme kommt.

Darüber hinaus war es Aufgabe der vorliegenden Erfindung eine Bräunungshilfe zur Verfügung zu stellen, die besonders pflegeleicht sind.

Eine weitere Aufgabe der Erfindung bestand darin, dass die Bräunungshilfen eine hohe Haltbarkeit, insbesondere eine hohe Beständigkeit gegen UV-Bestrahlung oder Bewitterung aufweisen.

Des weiteren lag der Erfindung die Aufgabe zu Grunde, Bräunungshilfen zur Verfügung zu stellen, die besonders einfach hergestellt werden können. So sollten zur Herstellung der Bräunungshilfen insbesondere Substrate verwendet werden können, die durch Extrusion, Spritzguss sowie durch Gussverfahren erhältlich sind.

Eine weitere Aufgabe der vorliegenden Erfindung bestand darin, Bräunungshilfen anzugeben, deren Sonnenschutz auf besonders einfache Weise eingestellt werden kann. So sollte die Aufenthaltsdauer unterhalb der Bräunungshilfe für viele Hauttypen auf eine vorgegebene Zeit eingestellt werden können. In Bezug auf die klassische Sonnenschutzcreme sollten diese Bräunungshilfen dem entsprechend auf einen bestimmten Sonnenschutzfaktor eingestellt werden können.

Darüber hinaus sollten die Bräunungshilfen der vorliegenden Erfindung besonders einfach an verschiedene Anforderungen und Anwendungen angepasst werden können. So sollten beispielsweise portable Schirme sowie fest eingebaute Bedachungen zur Verfügung gestellt werden. Hierbei sollten die Bräunungshilfen auf einfache Weise in Größe und Form den Anforderungen angepasst werden können.

Gelöst werden diese Aufgaben sowie weitere, die zwar nicht wörtlich genannt werden, sich aber aus den hierin diskutierten Zusammenhängen wie selbstverständlich ableiten lassen oder sich aus diesen zwangsläufig ergeben, durch die in Anspruch 1 beschriebenen Bräunungshilfen.

Zweckmäßige Abwandlungen der erfindungsgemäßen Bräunungshilfen werden in den auf Anspruch 1 rückbezogenen Unteransprüchen unter Schutz gestellt.

Hinsichtlich der Verwendung liefert Anspruch 16 eine Lösung der zu Grunde liegenden Aufgabe.

Dadurch, dass die Bräunungshilfe einen Polymethylmethacrylat-Formkörper umfasst, der 0,1 bis 1,5 Gew.-% eines 2,2,6,6-Tetraalkylpiperidyl-Gruppen umfassenden UV-Stabilisator enthält, der gelöst in MMA bei einer Konzentration von 1 Gew.-% bei 330 nm eine Transmission von mindestens 95 % zeigt und der Polymethylmethacrylat-Formkörper 0,005 bis 0,1 Gew.-% eines UV-Absorbers enthält, der gelöst in MMA bei einer Konzentration von 0,02 Gew.-% bei 320 nm eine Transmission von höchstens 5 % und bei 370 nm eine Transmission von mindestens 80 % zeigt, wobei die Transparenz des Polymethylmethacrylat-Formkörpers bei 380 nm mindestens 40 % beträgt, gelingt es überraschend eine Bräunungshilfe zur Verfügung zu stellen, mit Hilfe derer eine natürliche Bräunung mit Sonnenlicht möglich ist, ohne dass die Haut in Berührung mit Sonnenschutzcreme kommt.

Durch die erfindungsgemäßen Maßnahmen werden u. a. insbesondere folgende Vorteile erzielt:
➢ Durch die erfindungsgemäßen Bräunungshilfen ist ein Bräunen im Freien möglich.
➢ Des weiteren kann beim Bräunen auf energieintensive UV-Strahler verzichtet werden.
➢ Die erfindungsgemäßen Bräunungshilfen sind pflegeleicht sowie einfach herstellbar. So können insbesondere Formkörper verwendet werden, die durch Extrusion, Spritzguss sowie Gießverfahren erhältlich sind.
➢ Die Bräunungshilfen sind witterungsstabil und insbesondere gegenüber UV-Strahlung beständig. Des weiteren weisen die erfindungsgemäßen Bräunungshilfen sehr gute mechanische Eigenschaften auf.
➢ Des weiteren können die erfindungsgemäßen Bräunungshilfen einfach auf die unterschiedlichsten Anforderungen angepaßt werden. So können insbesondere transportable Schirme oder feste Bedachungen in jeglicher Größe hergestellt werden, um eine natürliche Bräunung der Haut zu ermöglichen, ohne daß hierdurch Schädigungen der Haut zu befürchten sind.
➢ Darüber hinaus können die Bräunungshilfen für jeden Hauttyp angefertigt werden, so dass unterschiedliche Aufenthaltsdauern im Freien zur Verfügung gestellt werden können.

Im Rahmen der vorliegenden Erfindung bedeutet der Begriff Bräunungshilfe eine Vorrichtung, die mindestens einen Polymethylmethacrylat-Formkörper umfasst, der zwischen das Sonnenlicht und eine zu bräunende Hautfläche gebracht werden kann. Dem entsprechend kann es sich hierbei insbesondere um transparente Bedachungen von Gebäuden oder um Schirme handeln, die beispielsweise immobil angebracht sind. Darüber hinaus sind Dächer, die beispielsweise auf Schiffen, insbesondere Tretbooten, Elektrobooten und ähnlichem angebracht werden können, als Bräunungshilfen geeignet.

Darüber hinaus kann es sich aber auch um transportable Schirme handeln, die je nach Dicke des Polymethylmethacrylat-Formkörpers in ihren Abmessungen fest sind, oder die auch zusammen geklappt werden können.

Die erfindungsgemäße Bräunungshilfe umfasst einen Polymethylmethacrylat-Formkörper. Polymethylmethacrylat (PMMA) ist in der Fachwelt an sich bekannt. Vorzugsweise umfasst der Polymethylmethacrylat-Formkörper mindestens 30 Gew.-%, bezogen auf das Gewicht des Polymethylmethacrylat-Formkörpers, Polymethylmethacrylat.

Polymethylmethacrylate werden im allgemeinen durch radikalische Polymerisation von Mischungen erhalten, die Methylmethacrylat enthalten. Im allgemeinen enthalten diese Mischungen mindestens 40 Gew.-%, vorzugsweise mindestens 60 Gew.-% und besonders bevorzugt mindestens 80 Gew.-%, bezogen auf das Gewicht der Monomere, Methylmethacrylat.

Daneben können diese Mischungen zur Herstellung von Polymethylmethacrylaten weitere (Meth)acrylate enthalten, die mit Methylmethacrylat copolymerisierbar sind. Der Ausdruck (Meth)acrylate umfasst Methacrylate und Acrylate sowie Mischungen aus beiden.

Diese Monomere sind weithin bekannt. Zu diesen gehören unter anderem (Meth)acrylate, die sich von gesättigten Alkoholen ableiten, wie beispielsweise Methylacrylat, Ethyl(meth)acrylat, Propyl(meth)acrylat, n-Butyl(meth)acrylat, tert.-Butyl(meth)acrylat, Pentyl(meth)acrylat und 2-Ethylhexyl(meth)acrylat;
(Meth)acrylate, die sich von ungesättigten Alkoholen ableiten, wie z. B. Oleyl(meth)acrylat, 2-Propinyl(meth)acrylat, Allyl(meth)acrylat, Vinyl(meth)acrylat;
Aryl(meth)acrylate, wie Benzyl(meth)acrylat oder Phenyl(meth)acrylat, wobei die Arylreste jeweils unsubstituiert oder bis zu vierfach substituiert sein können;
Cycloalkyl(meth)acrylate, wie 3-Vinylcyclohexyl(meth)acrylat, Bornyl(meth)acrylat;
Hydroxylalkyl(meth)acrylate, wie 3-Hydroxypropyl(meth)acrylat, 3,4-Dihydroxybutyl(meth)acrylat, 2-Hydroxyethyl(meth)acrylat, 2-Hydroxypropyl(meth)acrylat;
Glycoldi(meth)acrylate, wie 1,4-Butandiol(meth)acrylat, (Meth)acrylate von Etheralkoholen, wie Tetrahydrofurfuryl(meth)acrylat, Vinyloxyethoxyethyl(meth)acrylat; Amide und Nitrile der (Meth)acrylsäure, wie N-(3-Dimethylaminopropyl)(meth)acrylamid, N-(Diethylphosphono)(meth)acrylamid, 1-Methacryloylamido-2-methyl-2-propanol;
schwefelhaltige Methacrylate, wie Ethylsulfinylethyl(meth)acrylat, 4-Thiocyanatobutyl(meth)acrylat, Ethylsulfonylethyl(meth)acrylat, Thiocyanatomethyl(meth)acrylat, Methylsulfinylmethyl(meth)acrylat, Bis((meth)acryloyloxyethyl)sulfid;
mehrwertige (Meth)acrylate, wie Trimethyloylpropantri(meth)acrylat.

Neben den zuvor dargelegten (Meth)acrylaten können die zu polymerisierenden Zusammensetzungen auch weitere ungesättigte Monomere aufweisen, die mit Methylmethacrylat und den zuvor genannten (Meth)acrylaten copolymerisierbar sind.

Hierzu gehören unter anderem 1-Alkene, wie Hexen-1, Hepten-1; verzweigte Alkene, wie beispielsweise Vinylcyclohexan, 3,3-Dimethyl-1-propen, 3-Methyl-1-diisobutylen, 4-Methylpenten-1 ;
Acrylnitril; Vinylester, wie Vinylacetat;
Styrol, substituierte Styrole mit einem Alkylsubstituenten in der Seitenkette, wie z. B. α-Methylstyrol und α-Ethylstyrol, substituierte Styrole mit einem Alkylsubstitutenten am Ring, wie Vinyltoluol und p-Methylstyrol, halogenierte Styrole, wie beispielsweise Monochlorstyrole, Dichlorstyrole, Tribromstyrole und Tetrabromstyrole;
Heterocyclische Vinylverbindungen, wie 2-Vinylpyridin, 3-Vinylpyridin, 2-Methyl-5-vinylpyridin, 3-Ethyl-4-vinylpyridin, 2,3-Dimethyl-5-vinylpyridin, Vinylpyrimidin, Vinylpiperidin, 9-Vinylcarbazol, 3-Vinylcarbazol, 4-Vinylcarbazol, 1-Vinylimidazol, 2-Methyl-1-vinylimidazol,
N-Vinylpyrrolidon, 2-Vinylpyrrolidon, N-Vinylpyrrolidin, 3-Vinylpyrrolidin, N-Vinylcaprolactam, N-Vinylbutyrolactam, Vinyloxolan, Vinylfuran, Vinylthiophen, Vinylthiolan, Vinylthiazole und hydrierte Vinylthiazole, Vinyloxazole und hydrierte Vinyloxazole;
Vinyl- und Isoprenylether;
Maleinsäurederivate, wie beispielsweise Maleinsäureanhydrid, Methylmaleinsäureanhydrid, Maleinimid, Methylmaleinimid; und Diene, wie beispielsweise Divinylbenzol.

Im allgemeinen werden diese Comonomere in einer Menge von 0 bis 60 Gew.-%, vorzugsweise 0 bis 40 Gew.-% und besonders bevorzugt 0 bis 20 Gew.-%, bezogen auf das Gewicht der Monomeren, eingesetzt, wobei die Verbindungen einzeln oder als Mischung verwendet werden können.

Die Polymerisation wird im allgemeinen mit bekannten Radikalinitiatoren gestartet. Zu den bevorzugten Initiatoren gehören unter anderem die in der Fachwelt weithin bekannten Azoinitiatoren, wie AIBN und 1,1-Azobiscyclohexancarbonitril, sowie Peroxyverbindungen, wie Methylethylketonperoxid, Acetylacetonperoxid, Dilaurylperoxyd, tert.-Butylper-2-ethylhexanoat, Ketonperoxid, Methylisobutylketonperoxid, Cyclohexanonperoxid, Dibenzoylperoxid, tert.-Butylperoxybenzoat, tert.-Butylperoxyisopropylcarbonat, 2,5-Bis(2-ethylhexanoyl-peroxy)-2,5-dimethylhexan, tert.-Butylperoxy-2-ethylhexanoat, tert.-Butylperoxy-3,5,5-trimethylhexanoat, Dicumylperoxid, 1,1-Bis(tert.-butylperoxy)cyclohexan, 1,1-Bis(tert.-butylperoxy)3,3,5-trimethylcyclohexan, Cumylhydroperoxid, tert.-Butylhydroperoxid, Bis(4-tert.-butylcyclohexyl)peroxydicarbonat, Mischungen von zwei oder mehr der vorgenannten Verbindungen miteinander sowie Mischungen der vorgenannten Verbindungen mit nicht genannten Verbindungen, die ebenfalls Radikale bilden können.

Diese Verbindungen werden häufig in einer Menge von 0,01 bis 10 Gew.-%, vorzugsweise von 0,5 bis 3 Gew.-%, bezogen auf das Gewicht der Monomeren, eingesetzt.

Hierbei können verschiedene Poly(meth)acrylate eingesetzt werden, die sich beispielsweise im Molekulargewicht oder in der Monomerzusammensetzung unterscheiden.

Des weiteren kann der Polymethylmethacrylat-Formkörper weitere Polymere enthalten, um die Eigenschaften zu modifizieren. Hierzu gehören unter anderem Polyacrylnitrile, Polystyrole, Polyether, Polyester, Polycarbonate und Polyvinylchloride. Diese Polymere können einzeln oder als Mischung eingesetzt werden, wobei auch Copolymere, die von den zuvor genannten Polymere ableitbar sind.

Die erfindungsgemäßen Kunststoffsubstrate können beispielsweise aus Formmassen der zuvor genannten Polymere hergestellt werden. Hierbei werden im allgemeinen thermoplastische Formgebungsverfahren eingesetzt, wie Extrusion oder Spritzguss.

Das Gewichtsmittel des Molekulargewichts M_{w} der erfindungsgemäß als Formmasse zur Herstellung der Kunststoffsubstrate zu verwendenden Homo- und/oder Copolymere kann in weiten Bereichen schwanken, wobei das Molekulargewicht üblicherweise auf den Anwendungszweck und die Verarbeitungsweise der Formmasse abgestimmt wird. Im allgemeinen liegt es aber im Bereich zwischen 20 000 und 1 000 000 g/mol, vorzugsweise 50 000 bis 500 000 g/mol und besonders bevorzugt 80 000 bis 300 000 g/mol, ohne dass hierdurch eine Einschränkung erfolgen soll. Diese Größe kann beispielsweise mittels Gel-Permeations-Chromatographie bestimmt werden.

Des weiteren können die Kunststoffsubstrate durch Gusskammerverfahren erzeugt werden. Hierbei werden beispielsweise geeignete (Meth)acrylmischungen in einer Form gegeben und polymerisiert. Derartige (Meth)acrylmischungen weisen im allgemeinen die zuvor dargelegten (Meth)acrylate, insbesondere Methylmethacrylat auf. Des weiteren können die (Meth)acrylmischungen die zuvor dargelegten Copolymere sowie, insbesondere zur Einstellung der Viskosität, Polymere, insbesondere Poly(meth)acrylate, enthalten.

Das Gewichtsmittel des Molekulargewichts M_{w} der Polymere, die durch Gusskammerverfahren hergestellt werden, ist im allgemeinen höher als das Molekulargewicht von Polymeren, die in Formmassen verwendet werden. Hierdurch ergeben sich eine Reihe bekannter Vorteile. Im allgemeinen liegt das Gewichtsmittel des Molekulargewichts von Polymeren, die durch Gusskammerverfahren hergestellt werden im Bereich von 500 000 bis 10 000 000 g/mol, ohne dass hierdurch eine Beschränkung erfolgen soll.

Gemäss einer besonderen Ausführungsform der vorliegenden Erfindung weist die Matrix des Polymethylmethacrylat-Formkörpers mindestens 70, vorzugsweise mindestens 80 und besonders bevorzugt mindestens 90 Gew.-%, bezogen auf das Gewicht des Polymethylmethacrylat-Formkörpers, Polymethylmethacrylat auf.

Der Polymethylmethacrylat-Formkörper umfasst 0,1 bis 1,5, vorzugsweise von 0,3 bis 0,7 Gew.-%, bezogen auf das Gewicht des Polymethylmethacrylat-Formkörpers, eines 2,2,6,6-Tetraalkylpiperidyl-Gruppen umfassenden UV-Stabilisators, der gelöst in Methylmethacrylat bei einer Konzentration von 1 Gew.-%, bezogen die gesamte Mischung, bei 330 nm eine Transmission von mindestens 95 % zeigt. Derartige UV-Stabilisatoren sind an sich bekannt und kommerziell erhältlich.

Vorzugsweise handelt es sich hierbei um eine Verbindung gemäß Formel (I) 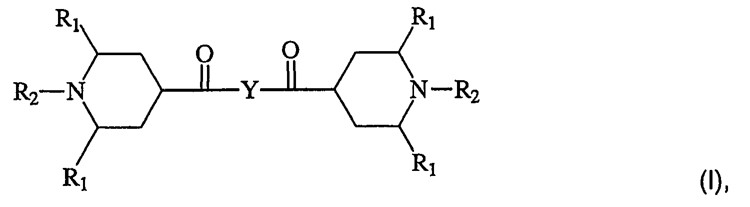 worin die Reste R₁ eine Alkylgruppe mit 1 bis 6 Kohlenstoffatome, R₂ ein Wasserstoffatom oder eine Alkylgruppe mit 1 bis 6 Kohlenstoffatome und Y eine verbindende Alkylengruppe mit 2 bis 20 Kohlenstoffatome darstellen.

Zu den bevorzugten Alkylgruppen gehören die Methyl-, Ethyl-, Propyl-, Isopropyl-, 1-Butyl-, 2-Butyl-, 2-Methylpropyl- und tert.-Butylgruppe.

Zu den bevorzugten verbindenden Alkylengruppen gehören Ethylen, Propylen, Butylen, Pentylen, Hexylen, Cyclohexylen, Heptylen, 2-Methylheptenylen, 3-Methylheptylen, Octylen, Nonylen, 3-Ethylnonylen, Decylen, Undecylen, 4-Propenylundecylen, Dodecylen, Tridecylen, Tetradecylen, Pentadecylen, Hexadecylen, Heptadecylen, Octadecylen, Nonadecylen.

Besonders bevorzugt ist der UV-Stabilisator Bis(2,2,6,6-tetramethyl-4-piperidyl)sebacat. Diese Verbindung ist kommerziell unter dem Handelsnamen TINUVIN 770 von Ciba Geigy, Mark LA 770 von Adeka Argus und Sanol LK 770 von erhältlich.

Des weiteren enthält der Polymethylmethacrylat-Formkörper 0,005 bis 0,1 Gew.-%, vorzugsweise 0,01 bis 0,04 Gew.-% bezogen auf das Gesamtgewicht des Polymethylmethacrylat-Formkörpers, mindestens eines UV-Absorbers, der gelöst in Methylmethacrylat bei einer Konzentration von 0,02 Gew.-%, bezogen auf das Gesamtgewicht der Mischung, bei 320 nm einer Transmission von höchstens 5 % und bei 370 nm einer Transmission von mindestens 80 % zeigt.

Bevorzugt sind UV-Stabilisatoren gemäß Formel (II) 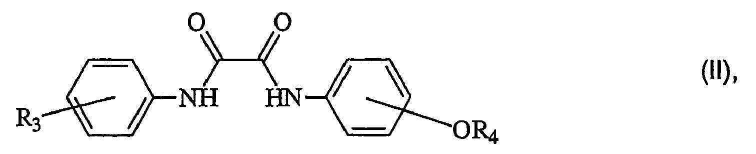 worin die Reste R₃ und R₄ unabhängig einen Alkyl- oder Cycloalkylrest mit 1 bis 20 Kohlenstoffatome darstellen. Die aliphatischen Reste können linear oder verzweigt sein, wobei diese Reste Substituenten, wie beispielsweise Halogenatome aufweisen können.

Zu den bevorzugten Alkylgruppen gehören die Methyl-, Ethyl-, Propyl-, Isopropyl-, 1-Butyl-, 2-Butyl-, 2-Methylpropyl-, tert.-Butylrest, Pentyl-, 2-Methylbutyl-, 1,1-Dimethylpropyl-, Hexyl-, Heptyl-, Octyl-, 1,1,3,3-Tetramethylbutyl, Nonyl-, 1-Decyl-, 2-Decyl-, Undecyl-, Dodecyl-, Pentadecyl- und die Eicosyl-Gruppe.

Zu den bevorzugten Cycloalkylgruppen gehören die Cyclopropyl-, Cyclobutyl-, Cyclopentyl-, Cyclohexyl-, Cycloheptyl- und die Cyclooctyl-Gruppe, die gegebenenfalls mit verzweigten oder nicht verzweigten Alkylgruppen substituiert sind.

Besonders bevorzugt wird die Verbindung der Formel (III) 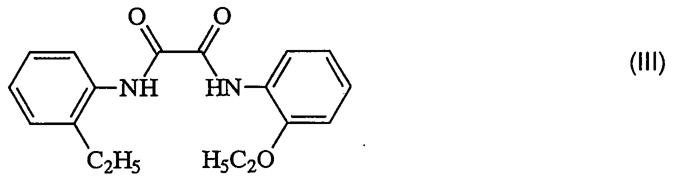 als UV-Absorber eingesetzt.

Diese Verbindung ist kommerziell von Clariant unter dem Handelsnamen ®Sanduvor VSU sowie von Ciba Geigy unter dem Handelsnamen ®Tinuvin 312 erhältlich.

Des weiteren kann der Polymethylmethacrylat-Formkörper weitere bekannte Additive umfassen, wobei deren Menge jedoch auf den Anwendungszweck der erfindungsgemäßen Bräunungshilfen eingeschränkt ist. Hierzu gehören unter anderem Antistatika, Antioxidantien, Entformungsmittel, Flammschutzmittel, Schmiermittel, Farbstoffe, Fliessverbesserungsmittel, Füllstoffe, Lichtstabilisatoren, UV-Absorber und organische Phosphorverbindungen, wie Phosphite oder Phosphonate, Pigmente, Verwitterungsschutzmittel und Weichmacher.

Zu den bevorzugten Additiven gehören Farbstoffe, die gelöst in Methylmethacrylat bei einer Konzentration von 0,01 Gew.-% bei 350 nm eine Transmission von mindestens 30% zeigen. Derartige Farbstoffe sind an sich bekannt und beispielsweise unter dem Handelsnamen ®Makrolex blau RR, ®Makrolex violett B, ®Makrolex violett 3R, ®Makrolex grün 5B, ®Makrolex grün G, von Bayer, ®Sandoplast bau 2B, ®Sandoplast rot BB, sowie ®Sandoplast grün G von Clariant, ®Mikrolitviol B-K von Ciba erhältlich.

Des weiteren gehören zu den bevorzugten Additiven IR-Absorber. Zu diesen gehören unter anderem IR-Absorber 2052 von Bayer.

Des weiteren kann der Polymethylmethacrylat-Formkörper sphärische Partikel umfassen. Der Begriff sphärisch bezeichnet im Rahmen der vorliegenden Erfindung, dass die Partikel vorzugsweise eine kugelförmige Gestalt aufweisen, wobei dem Fachmann offensichtlich ist, dass aufgrund der Herstellungsmethoden auch Partikel mit anderer Gestalt enthalten sein können, oder dass die Form der Partikel von der idealen Kugelgestalt abweichen kann.

Dementsprechend bedeutet der Begriff sphärisch, dass das Verhältnis von der grössten Ausdehnung der Partikel zur geringsten Ausdehnung maximal 4, vorzugsweise maximal 2 beträgt, wobei diese Ausdehnungen jeweils durch den Schwerpunkt der Partikel gemessen werden. Vorzugsweise sind mindestens 70%, besonders bevorzugt mindestens 90%, bezogen auf die Zahl der Partikel, sphärisch.

Die Partikel haben vorzugsweise einen mittleren Durchmesser (Gewichtsmittel) im Bereich von 5 bis 50 µm, vorzugsweise im Bereich von 8 bis 25 µm. Günstigerweise liegen 75% der Partikel im Bereich von 5 bis 35 µm.

Diese Partikel können beispielsweise aus anorganischen Materialien, insbesondere aus BaSO₄ oder Kunststoff sein, wobei Kunststoff partikel bevorzugt sind. Hierbei weist der Brechungsindex der Partikel eine bei der Na-D-Linie (589 nm) und bei 20°C gemessene Brechzahl nₒ auf, die um 0,02 bis 0,2 Einheiten von der Brechzahl nₒ des Matrixkunststoffs unterscheidet.

Vorzugsweise umfassen die sphärische Kunststoffpartikel vernetzes Polystyrol und/oder vernetzte Poly(meth)acrylate.

Besonders bevorzugt weisen Mischungen aus denen die Kunststoffpartikel hergestellt werden mindestens 80 Gew.-% Styrol und mindestens 0,5 Gew.-% Divinylbenzol auf.

Die Herstellung von vernetzten Kunststoffpartikeln ist in der Fachwelt bekannt. So können die Streupartikel durch Emulsionspolymerisation hergestellt werden, wie beispielsweise in EP-A 342 283 oder EP-A 269 324 beschrieben, ganz besonders bevorzugt durch Polymerisation in organischer Phase, wie beispielsweise in der deutschen Patentanmeldung P 43 27 464.1 beschrieben, wobei bei der letztgenannten Polymerisationstechnik besonders enge Teilchengrößenverteilungen oder anders ausgedruckt besonders geringe Abweichungen der Teilchendurchmesser vom mittleren Teilchendurchmesser auftreten.

Der Polymethylmethacrylat-Formkörper kann beispielsweise 2 bis 50 Gew.-%, vorzugsweise 4 bis 10 Gew.-% Partikel umfassen, die eine Streuwirkung entfalten.

Hierdurch kann überraschend eine Verringerung der Blendwirkung durch Sonneneinstrahlung erzielt werden, ohne dass die Bräunung der Haut allzu sehr beeinträchtigt wird. Diese bevorzugte Ausführungsform der erfindungsgemäßen Bräunungshilfe eignet sich insbesondere bei Anwendungen, bei denen eine Blendwirkung der Sonne verringert werden soll.

Gemäß einem besonderen Aspekt der vorliegenden Erfindung weist der Polymethylmethacrylat-Formkörper eine Schlagzähigkeit von mindestens 20 kJ/m² gemessen gemäß DIN 53453 (Normstab) auf. Zur Verbesserung dieser mechanischen Eigenschaft können insbesondere bekannte Schlagzähmodifier eingesetzt werden.

Bevorzugte schlagzähe Formmassen, die zur Herstellung des Polymethylmethacrylat-Formkörper dienen können, enthalten 1 bis 30, bevorzugt 2 bis 20, besonders bevorzugt 3 bis 15, insbesondere 5 bis 12 Gew.-% eines Schlagzähmodifizierungsmittels, welches eine Elastomerphase aus vernetzten Polymerisatteilchen darstellt.

Das Schlagzähmodifizierungsmittel kann in an sich bekannter Weise durch Perlpolymerisation oder durch Emulsionspolymerisation erhalten werden.

Bevorzugte Schlagzähmodifizierungsmittel stellen vernetzte Teilchen mit einer mittleren Teilchengröße im Bereich von 50 bis 1000 nm, bevorzugt 60 bis 500 nm und besonders bevorzugt 80 bis 120 nm dar.

Derartige Partikel können beispielsweise durch die radikalische Polymerisation von Mischungen erhalten werden, die in der Regel mindestens 40 Gew.-%, bevorzugt 50 bis 70 Gew.-% Methylmethacrylat, 20 bis 80 Gew.-%, bevorzugt 25 bis 35 Gew.-% Butylacrylat sowie 0,1 bis 2, bevorzugt 0,5 bis 1 Gew.-% eines vernetzenden Monomeren, z. B. einem mehrfunktionellen (Meth)acrylat, wie z. B. Allylmethacrylat und Comonomeren, die mit den zuvor genannten Vinylverbindungen copolymerisiert werden können.

Zu den bevorzugten Comonomeren gehören unter anderem C₁-C₄-Alkyl(meth)acrylaten, wie Ethylacrylat oder Butylmethacrylat, bevorzugt Methylacrylat, oder anderen vinylisch polymerisierbaren Monomeren wie z. B. Styrol. Die Mischungen zur Herstellung der zuvor genannten Partikel können vorzugsweise 0 bis 10, bevorzugt 0,5 bis 5 Gew.-% Comonomere umfassen.

Besonders bevorzugte Schlagzähmodifizierungsmittel sind Polymerisatteilchen, die einen zwei-, besonders bevorzugt einen dreischichtigen Kern-Schale-Aufbau aufweisen. Derartige Kern-Schale-Polymerisate sind unter anderem in EP-A 0 113 924, EP-A 0 522 351, EP-A 0 465 049 und EP-A 0 683 028 beschrieben.

Besonders bevorzugte Schlagzäh-Modifier auf Basis von Acrylatkautschuk haben unter anderem folgenden Aufbau:
- Kern:: Polymerisat mit einem Methylmethacrylatanteil von mindestens 90 Gew.-%, bezogen auf das Gewicht des Kerns.
- Schale 1:: Polymerisat mit einem Butylacrylatanteil von mindestens 80 Gew.-%, bezogen auf das Gewicht der ersten Schale.
- Schale 2:: Polymerisat mit einem Methylmethacrylatanteil von mindestens 90 Gew.-%, bezogen auf das Gewicht der zweiten Schale.

Der Kern sowie die Schalen können neben den genannten Monomeren jeweils weitere Monomere enthalten. Diese wurden zuvor dargelegt, wobei besonders bevorzugte Comonomere vernetzend wirken.

Beispielsweise kann ein bevorzugter Acrylatkautschuk-Modifier folgenden Aufbau aufweisen:
- Kern:: Copolymerisat aus Methylmethacrylat (95,7 Gew.-%), Ethylacrylat (4 Gew.-%) und Allylmethacrylat (0,3 Gew.-%)
- S1:: Copolymerisat aus Butylacrylat (81,2 Gew.-%), Styrol (17,5 Gew.-%) und Allylmethacrylat (1,3 Gew.-%)
- S2:: Copolymerisat aus Methylmethacrylat (96 Gew.-%) und Ethylacrylat (4 Gew.-%)

Das Verhältnis von Kern zu Schale(n) der Acrylatkautschuk-Modifier kann in weiten Bereichen schwanken. Vorzugsweise liegt das Gewichtsverhältnis Kern zu Schale K/S im Bereich von 20:80 bis 80:20, bevorzugt von 30:70 zu 70:30 bis Modifiern mit einer Schale bzw. das Verhältnis von Kern zu Schale 1 zu Schale 2 K/S1/S2 im Bereich von 10:80:10 bis 40:20:40, besonders bevorzugt von 20:60:20 bis 30:40:30 bei Modifiern mit zwei Schalen.

Die Partikelgröße der Kern-Schale-Modifier liegt üblich im Bereich von 50 bis 1000 nm, vorzugsweise 100 bis 500 nm und besonders bevorzugt von 150 bis 450 nm, ohne daß hierdurch eine Beschränkung erfolgen soll.

Gemäß einer besonderen Ausführungsform weist der Polymethylmethacrylat-Formkörper ein E-Modul von mindestens 2800 N/mm², vorzugsweise mindestens 3300 N/mm² gemäß ISO 527/2 auf.

Die Dicke des Polymethylmethacrylat-Formkörpers, kann je nach Anwendungszweck in einem weiten Bereich liegen. Im allgemeinen weist dieser Formkörper eine Dicke im Bereich von 1 bis 200 mm vorzugsweise 2 bis 20 mm auf.

Die Oberfläche der Bräunungshilfen kann glänzend oder matt erscheinen. Gemäß einer besonderen Ausführungsform können die Bräunungshilfen mit einer Satinoberfläche ausgestattet sein.

Die Größe der Bräunungshilfe kann den Anforderungen angepaßt werden. So können Dächer beispielsweise mehrere 100 m² groß sein oder kleinere Schirme lediglich 1 bis 2 m² umfassen.

Nachfolgend wird die Erfindung durch Beispiele und Vergleichsbeispiele eingehender erläutert, ohne das die Erfindung auf diese Beispiele beschränkt werden soll.

### Beispiel 1

Eine Polymethylmethacrylat-Platte mit einer Dicke von 3mm wurde in einem Gussverfahren hergestellt. Hierzu wurden 994,208 g Sirup (Mischung aus Methylmethacrylat/ Polymethylmethacrylat mit 8 bis 10 % Umsatz), 0,039 g ®Mikrolit Violett BK, 0,03 g Solvapermrot BB, 0,200 g ®Sanduvor VSU, erhältlich von Clariant, 5 g ®Tinuvin 770 DF erhältlich von Ciba Geigy und 0,55 g 2,2-Azodi(isobutyronitril) (AIBN, von Akzo Nobel gemischt und in eine Gussglasform gegossen. Diese Form wurde 150 Minuten auf 77°C erhitzt.

Die so erhaltene Platte zeigte eine UV-Durchlässigkeit bei 380 nm von 74,42% und bei 330 nm eine Transmission von 4,85%. Die berechnete effektive Bestrahlungsstärke betrug 12,2 mW/m².

Die effektive Bestrahlungsstärke lässt sich durch die Erythemfunktion berechnen, die die Effizienz von Ultraviolettstrahlung zur Erzeugung einer Rötung einer menschlichen Haut ausgedrückt wird. Die Bewertung erfolgt durch Multiplikation des durchgelassenen Bestrahlungsspektrums mit der Erythemfunktion.

Das Bestrahlungsspektrum ergibt sich aus dem Sonnenspektrum gemäß Norm DIN 67501. Durch Integration des bewerteten Spektrums über die Wellenlänge erhält man die effektive Bestrahlungsstärke, wobei sich die Effektivität auf die Wirksamkeit zur Erzeugung eines Sonnenbrandes bezieht. Das ursprüngliche Sonnenspektrum weist eine effektive Bestrahlungsstärke von ca. 250,9 mW/m² auf.

### Beispiel 2

Beispiel 1 wurde im wesentlichen wiederholt, wobei jedoch eine Mischung eingesetzt wurde, die 975,0616 g Sirup, 0,20g ®Sanduvor VSU, 5,00 g ®Tinuvin 770 DF und 0,5g AIBN und 19,2384 g einer Mischung umfasst, die aus 8,64173 g ®Makrolex Orange 3G, 15,58967 g ®Makrolex Violett 3R, 238,70890 g ®Mikrolit Grün GK 10 %, 1,32260 g ®Termoplastgelb R 154, 9735,73170 g Pastengrundlage, jeweils bezogen auf 10 000 g, besteht. Die Pastengrundlage besteht aus 83,25 Gew.-% Dioctylphthalat, 1,25 % Catafor CA 100, 5,5 % Rizinusöl 10 % Aerosil R 972.

Die so erhaltene Platte zeigte eine UV-Durchlässigkeit bei 380 nm von 46,19% und bei 330 nm eine Transmission von 0,06%. Die berechnete effektive Bestrahlungsstärke betrug 4,6 mW/m².

### Beispiel 3

Das Beispiel 2 wurde im wesentlichen wiederholt, wobei jedoch eine Mischung eingesetzt wurde, die 868,80g MMA, 120,00g ®Plex 4065 erhältlich von Röhm GmbH & Co. KG, 5,50g Sandoplastblau 2G 1%ig erhältlich von Clariant, 0,20g ®Sanduvor VSU, 5,00 g ®Tinuvin 770 DF und 0,5g AIBN enthielt.

Die so erhaltene Platte zeigte eine UV-Durchlässigkeit bei 380 nm von 51,18% und bei 330 nm eine Transmission von 3,34%. Die berechnete effektive Bestrahlungsstärke betrug 8,5 mW/m².

## Patentansprüche

1. Bräunungshilfe, **dadurch gekennzeichnet, dass** die Bräunungshilfe einen Polymethylmethacrylat-Formkörper umfasst, der 0,1 bis 1,5 Gew.-% eines 2,2,6,6-Tetraalkylpiperidyl-Gruppen umfassenden UV-Stabilisators enthält, der gelöst in MMA bei einer Konzentration von 1 Gew.-% bei 330nm eine Transmission von mindestens 95% zeigt und der Polymethylmethacrylat-Formkörper 0,005 bis 0,1 Gew.-% eines UV-Absorbers enthält, der gelöst in MMA bei einer Konzentration von 0,02 Gew.-% bei 320nm eine Transmission von höchstens 5% und bei 370 nm eine Transmission von mindestens 80% zeigt, wobei die Transparenz des Polymethylmethacrylat-Formkörpers bei 380nm mindestens 40% beträgt.

2. Bräunungshilfe gemäß Anspruch 1, **dadurch gekennzeichnet, dass** der UV-Stabilisator eine Verbindung gemäß Formel (I) ist 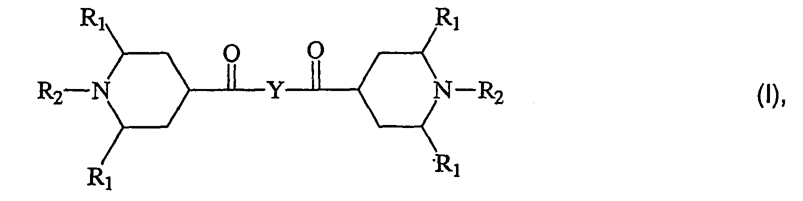 worin die Reste R₁ eine Alkylgruppe mit 1 bis 6 Kohlenstoffatome, R₂ ein Wasserstoffatom oder eine Alkylgruppe mit 1 bis 6 Kohlenstoffatome und Y eine verbindende Alkylengruppe mit 2 bis 20 Kohlenstoffatome darstellen.

3. Bräunungshilfe gemäß Anspruch 2, **dadurch gekennzeichnet, dass** der UV-Stabilisator Bis(2,2,6,6-tetramethyl-4-piperidyl)sebacat ist.

4. Bräunungshilfe gemäß einem der vorhergehenden Ansprüche, **dadurch gekennzeichnet, dass** der UV-Stabilisator eine Verbindung gemäß Formel (II) ist 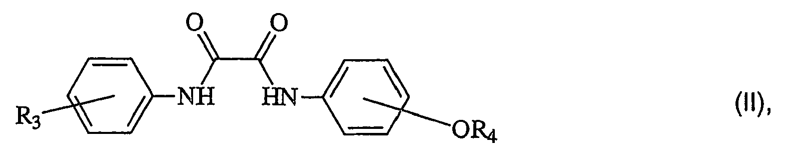 worin die Reste R₃ und R₄ unabhängig einen Alkylrest mit 1 bis 20 Kohlenstoffatome darstellen.

5. Bräunungshilfe gemäß einem der vorhergehenden Ansprüche, **dadurch gekennzeichnet, dass** der UV-Absorber die Verbindung gemäß Formel (III) ist

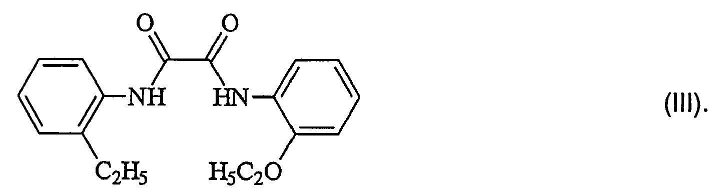

6. Bräunungshilfe gemäß einem der vorhergehenden Ansprüche, **dadurch gekennzeichnet, dass** der Kunststoffformkörper zusätzlich Farbstoffe umfasst, die gelöst in MMA bei einer Konzentration von 0,01 Gew.-% bei 350nm eine Transmission von mindestens 30% zeigen.

7. Bräunungshilfe gemäß einem der vorhergehenden Ansprüche, **dadurch gekennzeichnet, dass** die Transparenz des Polymethylmethacrylat-Formkörper bei 400 nm mindestens 30% beträgt.

8. Bräunungshilfe gemäß einem der vorhergehenden Ansprüche, **dadurch gekennzeichnet, dass** die Transparenz des Polymethylmethacrylat-Formkörper bei 330 nm höchstens 30% beträgt.

9. Bräunungshilfe gemäß einem der vorhergehenden Ansprüche, **dadurch gekennzeichnet, dass** das Verhältnis von Transparenz des Polymethylmethacrylat-Formkörper bei 400 nm zur Transparenz bei 330nm mindestens 20 beträgt.

10. Bräunungshilfe gemäß einem der vorhergehenden Ansprüche, **dadurch gekennzeichnet, dass** der Polymethylmethacrylat-Formkörper einen IR-Absorber umfasst.

11. Bräunungshilfe gemäß einem oder mehreren der vorhergehenden Ansprüche, **dadurch gekennzeichnet, dass** der Polymethylmethacrylat-Formkörper eine Schlagzähigkeit von mindestens 20 kJ/m² aufweist.

12. Bräunungshilfe gemäß einem oder mehreren der vorhergehenden Ansprüche, **dadurch gekennzeichnet, dass** der Polymethylmethacrylat-Formkörper eine Dicke im Bereich von 1 bis 200 mm aufweist.

13. Bräunungshilfe gemäß einem oder mehreren der vorhergehenden Ansprüche, **dadurch gekennzeichnet, dass** der Polymethylmethacrylat-Formkörper ein E-Modul von mindestens 2800 N/mm² aufweist.

14. Bräunungshilfe gemäß einem der vorhergehenden Ansprüche, **dadurch gekennzeichnet, dass** der Polymethylmethacrylat-Formkörper sphärische Partikel umfasst.

15. Verwendung von Polymethylmethacrylat-Formkörper mit einer Transparenz bei 380 nm von mindestens 40% als Bräunungshilfe.

## Claims

1. Tanning aid, **characterized in that** the tanning aid comprises a polymethyl methacrylate shaped body which comprises 0.1 to 1.5% by weight of a UV stabilizer containing 2,2,6,6-tetraalkylpiperidyl groups and which, dissolved in MMA at a concentration of 1% by weight, exhibits a transmission of at least 95% at 330 nm and the polymethyl methacrylate shaped body comprises 0.005 to 0.1% by weight of a UV absorber which, dissolved in MMA at a concentration of 0.02% by weight, exhibits a transmission of at most 5% at 320 nm and a transmission of at least 80% at 370 nm, where the transparency of the polymethyl methacrylate shaped body at 380 nm is at least 40%.

2. Tanning aid according to Claim 1, **characterized in that** the UV stabilizer is a compound according to formula (I) 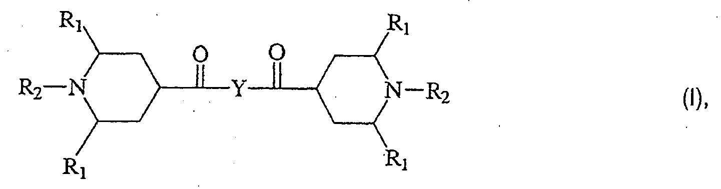 in which the radicals R₁ are an alkyl group having 1 to 6 carbon atoms, R₂ is a hydrogen atom or an alkyl group having 1 to 6 carbon atoms and Y is a linking alkylene group having 2 to 20 carbon atoms.

3. Tanning aid according to Claim 2, **characterized in that** the UV stabilizer is bis(2,2,6,6-tetramethyl-4-piperidyl) sebacate.

4. Tanning aid according to one of the preceding claims, **characterized in that** the UV stabilizer is a compound according to formula (II) 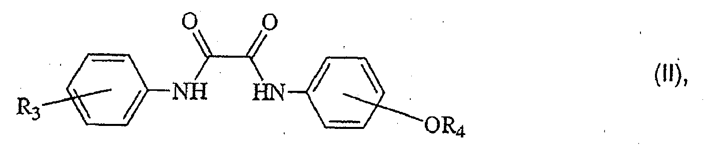 in which the radicals R₃ and R₄, independently, are an alkyl radical having 1 to 20 carbon atoms.

5. Tanning aid according to one of the preceding claims, **characterized in that** the UV absorber is the compound according to formula (III)

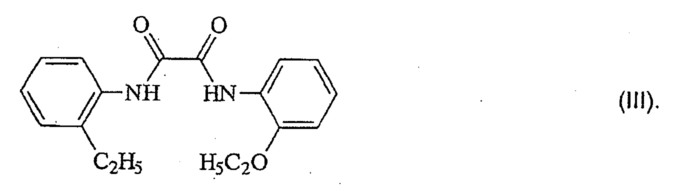

6. Tanning aid according to one of the preceding claims, **characterized in that** the plastic shaped body additionally comprises dyes which, dissolved in MMA at a concentration of 0.01% by weight, exhibit a transmission of at least 30% at 350 nm.

7. Tanning aid according to one of the preceding claims, **characterized in that** the transparency of the polymethyl methacrylate shaped body at 400 nm is at least 30%.

8. Tanning aid according to one of the preceding claims, **characterized in that** the transparency of the polymethyl methacrylate shaped body at 330 nm is at most 30%.

9. Tanning aid according to one of the preceding claims, **characterized in that** the ratio of transparency of the polymethyl methacrylate shaped body at 400 nm to the transparency at 330 nm is at least 20.

10. Tanning aid according to one of the preceding claims, **characterized in that** the polymethyl methacrylate shaped body comprises an IR absorber.

11. Tanning aid according to one or more of the preceding claims, **characterized in that** the polymethyl methacrylate shaped body has an impact resistance of at least 20 kJ/m².

12. Tanning aid according to one or more of the preceding claims, **characterized in that** the polymethyl methacrylate shaped body has a thickness in the range from 1 to 200 mm.

13. Tanning aid according to one or more of the preceding claims, **characterized in that** the polymethyl methacrylate shaped body has an E modulus of at least 2800 N/mm².

14. Tanning aid according to one of the preceding claims, **characterized in that** the polymethyl methacrylate shaped body comprises spherical particles.

15. Use of polymethyl methacrylate shaped bodies having a transparency at 380 nm of at least 40% as tanning aid.

## Revendications

1. Produit d'aide au bronzage, **caractérisé en ce que** le produit d'aide au bronzage comprend un corps façonné en poly(méthacrylate de méthyle), qui contient 0,1 à 1,5% en poids d'un stabilisateur des UV comprenant des groupes 2,2,6,6-tétraalkylpipéridyle qui présente, lorsqu'il est dissous dans du MMA en une concentration de 1% en poids à 330 nm, une transmission d'au moins 95% et le corps façonné en poly(méthacrylate de méthyle) contient 0,005 à 0,1% en poids d'un absorbant des UV qui présente, lorsqu'il est dissous dans du MMA en une concentration de 0,02% en poids à 320 nm, une transmission d'au plus 5% et, à 370 nm, une transmission d'au moins 80%, la transparence du corps façonné en poly(méthacrylate de méthyle) à 380 nm étant d'au moins 40%.

2. Produit d'aide au bronzage selon la revendication 1, **caractérisé en ce que** le stabilisateur des UV est un composé selon la formule (I) 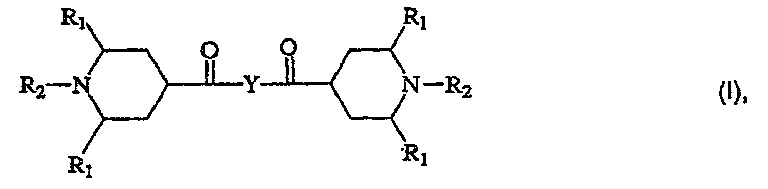 où les radicaux R₁ représentent un groupe alkyle comprenant 1 à 6 atomes de carbone, les radicaux R₂ représentent un atome d'hydrogène ou un groupe alkyle comprenant 1 à 6 atomes de carbone et Y représente un groupement alkylène de liaison comprenant 2 à 20 atomes de carbone.

3. Produit d'aide au bronzage selon la revendication 2, **caractérisé en ce que** le stabilisateur des UV est le sébacate de bis(2,2,6,6-tétraméthyl-4-pipéridyle).

4. Produit d'aide au bronzage selon l'une quelconque des revendications précédentes, **caractérisé en ce que** le stabilisateur des UV est un composé selon la formule (II) 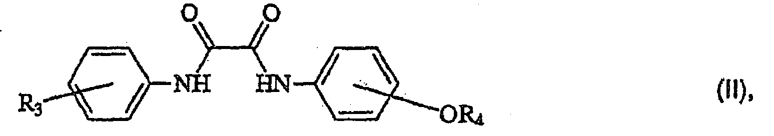 dans laquelle les radicaux R₃ et R₄ représentent, indépendamment l'un de l'autre un radical alkyle comprenant 1 à 20 atomes de carbone.

5. Produit d'aide au bronzage selon l'une quelconque des revendications précédentes, **caractérisé en ce que** l'absorbant des UV est un composé selon la formule (III)

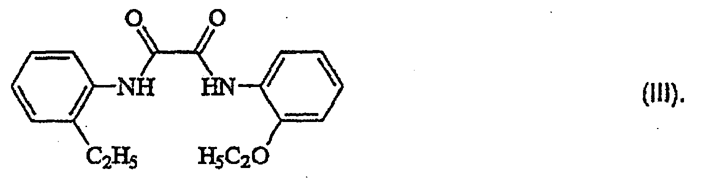

6. Produit d'aide au bronzage selon l'une quelconque des revendications précédentes, **caractérisé en ce que** le corps façonné en matériau synthétique comprend en outre des colorants qui présentent, lorsqu'ils sont dissous dans du MMA en une concentration de 0,01% en poids à 350 nm, une transmission d'au moins 30%.

7. Produit d'aide au bronzage selon l'une quelconque des revendications précédentes, **caractérisé en ce que** la transparence du corps façonné en poly(méthacrylate de méthyle) à 400 nm est d'au moins 30%.

8. Produit d'aide au bronzage selon l'une quelconque des revendications précédentes, **caractérisé en ce que** la transparence du corps façonné en poly(méthacrylate de méthyle) à 330 nm est d'au plus 30%.

9. Produit d'aide au bronzage selon l'une quelconque des revendications précédentes, **caractérisé en ce que** le rapport de la transparence du corps façonné en poly(méthacrylate de méthyle) à 400 nm à la transparence à 330 nm est d'au moins 20.

10. Produit d'aide au bronzage selon l'une quelconque des revendications précédentes, **caractérisé en ce que** le corps façonné en poly(méthacrylate de méthyle) comprend un absorbant des IR.

11. Produit d'aide au bronzage selon l'une ou plusieurs des revendications précédentes, **caractérisé en ce que** le corps façonné en poly(méthacrylate de méthyle) présente une résilience d'au moins 20 kJ/m².

12. Produit d'aide au bronzage selon l'une ou plusieurs des revendications précédentes, **caractérisé en ce que** le corps façonné en poly(méthacrylate de méthyle) présente une épaisseur dans la plage de 1 à 200 mm.

13. Produit d'aide au bronzage selon l'une ou plusieurs des revendications précédentes, **caractérisé en ce que** le corps façonné en poly(méthacrylate de méthyle) présente un module E d'au moins 2800 N/mm².

14. Produit d'aide au bronzage selon l'une quelconque des revendications précédentes, **caractérisé en ce que** le corps façonné en poly(méthacrylate de méthyle) comprend des particules sphériques.

15. Utilisation de corps façonnés en poly(méthacrylate de méthyle) présentant une transparence à 380 nm d'au moins 40% comme produits d'aide au bronzage.
